# EUROPEAN PATENT APPLICATION

(11) **EP 1 997 509 A1**
(43) Date of publication of application: **03.12.2008**
(21) Application number: 07010912.9
(22) Date of filing: 01.06.2007
(51) Int. Cl.: A61K 38/48

(54) **Process for providing a temperature-stable muscle relaxant on the basis of the neurotoxic component of botulinum toxin**

(71) Applicant: Merz Pharma GmbH & Co. KGaA, 60318 Frankfurt (DE)
(72) Inventor: Grein, Dr. Swen, 61118 Bad Vilbel (DE); Mander, Dr. Gerd J., 61169 Friedberg (DE); Marx, Dr. Matthias, 68199 Mannheim (DE)
(74) Representative: Ricker, Mathias

(57) **Abstract**

The present invention relates to a process for providing a muscle relaxant which is stable at temperatures above 20°C, wherein said muscle relaxant is a solid dry composition comprising the neurotoxic component of botulinum toxin free of complexing proteins.

## Description

### FIELD OF THE INVENTION

The present invention provides a process for providing a muscle relaxant at a temperature above 20°C, wherein said muscle relaxant is a solid dry composition comprising the neurotoxic component of botulinum toxin free of complexing proteins.

### BACKGROUND OF THE INVENTION

Botulinum toxin is produced by the bacterium *Clostridium.* There are seven antigenically distinct serotypes of botulinum toxin, namely botulinum toxin A, B, C, D, E, F and G. Botulinum toxins are released from lysed Clostridium cultures generally in the form of a complex, i.e. the sub-unit responsible for the toxic properties of the Botulinum toxin (the so-called "neurotoxic component"), is associated with other bacterial proteins, which together form a toxin complex. The molecular weight of this complex may vary from about 300,000 to about 900,000 Da. The complexing proteins are, for example, various hemagglutinins. The proteins of this toxin complex are not toxic themselves but are believed to provide stability to the neurotoxic component and are responsible for oral toxicity in botulinum intoxications. Unlike the toxin complex, the neurotoxic component in its isolated and pure form, i.e. devoid of any complexing Clostridium proteins, is acid labile and does not resist the aggressive environment in the gastrointestinal tract.

The neurotoxic component of the botulinum toxin complex is initially formed as a single polypeptide chain, having in the case of serotype A a molecular weight of approximately 150 kDa. In other serotypes the neurotoxic component has been observed to vary between about 145 and about 170 kDa, depending on the bacterial source. In the case of serotype A, for example, proteolytic processing of the polypeptide results in an activated polypeptide in the form of a dichain polypeptide consisting of a heavy chain and a light chain, which are linked by a disulfide bond. In humans, the heavy chain mediates binding to pre-synaptic cholinergic nerve terminals and internalization of the toxin into the cell. The light chain is believed to be responsible for the toxic effects, acting as zink-endopeptidase and cleaving specific proteins responsible for membrane fusion (SNARE complex) (see e.g. Montecucco C., Shiavo G., Rosetto O: The mechanism of action of tetanus and botulinum neurotoxins. Arch Toxicol. 1996; 18 (Suppl.): 342-354)).

The neurotoxic subunit of the Botulinum toxin complex is referred herein as the "neurotoxic component" or the "neurotoxic component free of complexing proteins".

The terms "botulinum toxin" or "botulinum toxins" as used throughout the present applicaton, refer to the neurotoxic component devoid of any other clostridial proteins, but also to the "botulinum toxin complex": The term "botulinum toxin" is used herein in cases when no discrimination between the complex or the neurotoxic component is necessary or desired. The complex usually contains additional, so-called "non-toxic" proteins, which we will refer to as "complexing proteins" or "bacterial proteins".

Despite its toxic effects, botulinum toxin complex has been used as a therapeutic agent in a large number of diseases. Botulinum toxin serotype A was approved for human use in the United States in 1989 for the treatment of strabism, blepharospasm, and other disorders. It is commercially available as Botulinum toxin A protein complex, for example, under the tradename BOTOX (Allergan Inc) or under the tradename DYSPORT (Ipsen Ltd). For therapeutic application the complex is injected directly into the muscle to be treated. At physiological pH, the toxin is released from the protein complex and the desired pharmacological effect takes place.

A pharmaceutical composition comprising the neurotoxic component of botulinum toxin type A in isolated form is commercially available in Germany from Merz Pharmaceuticals GmbH under the trademark Xeomin^{®}. The production of the neurotoxic component of botulinum toxin type A and B are described, for example, in the international patent application WO 00/74703.

Before administering it to a patient, typically intramuscular directly into the affected muscle, said composition is dissolved in physiological saline solution.

With regard to the composition and dosing of the medicament on the basis of botulinum toxin, and in regard to the composition, dosing and frequency of administration of the medicament on the basis of the neurotoxic component of botulinum toxin, reference is made to US 60/817 756.

In addition to the above-recited function of the complexing proteins it has been speculated that they also protect the neurotoxic component of the Botulinum toxin complex from harsh environmental conditions, and that the neurotxic component as such is highly susceptible to degradation and/or inactivation, especially when subjected to short-term temperature stress, such as storage and/or transport in warm to hot climate or during summer in general.

For said reason, utmost care is generally taken in the past to prevent the medicaments on the basis of the Botulinum toxins and those on the basis of the neurotoxic component of Botulinum toxin in particular, from reaching a temperature of above 4°C, e.g. close to 20°C. In most cases the vials containing the solid dry composition comprising the botulinum toxins were stored frozen (around -20°C), under ice or at least in a refrigerator (around 4°C). The necessary cooling means added to the cost of providing the medicaments.

In view of the above situation, the applicants undertook studies regarding the temperature stability of muscle relaxants on the basis of botulinum toxin. It was surprisingly found that the neurotoxic component of botulinum toxin is significantly more temperature stable than expected in the art. The hereinunder described invention is based on this finding.

### SUMMARY OF THE INVENTION

The present invention relates to a process for providing a muscle relaxant at temperatures above 20°C, wherein said muscle relaxant is a solid dry composition comprising the neurotoxic component of botulinum toxin free of complexing proteins, preferably said process involves storage and/or transport. In another preferred embodiment, said provision is a step within a process for preparing said muscle relaxant, more preferably a step carried out after the proteins including the neurotoxic component of botulinum toxin have been dried.

### BRIEF DESCRIPTION OF THE DRAWINGS

Fig. 1 shows the impact of temperature on the biological activity of Xeomin® after storage at 60°C for a period of up to 30 days; the biological activity was tested at the indicated points in time;
Fig. 2 shows the impact of temperature on the biological activity of Xeomin® after storage at 70°C for a period of up to 10 days; the biological activity was tested at the indicated points in time;

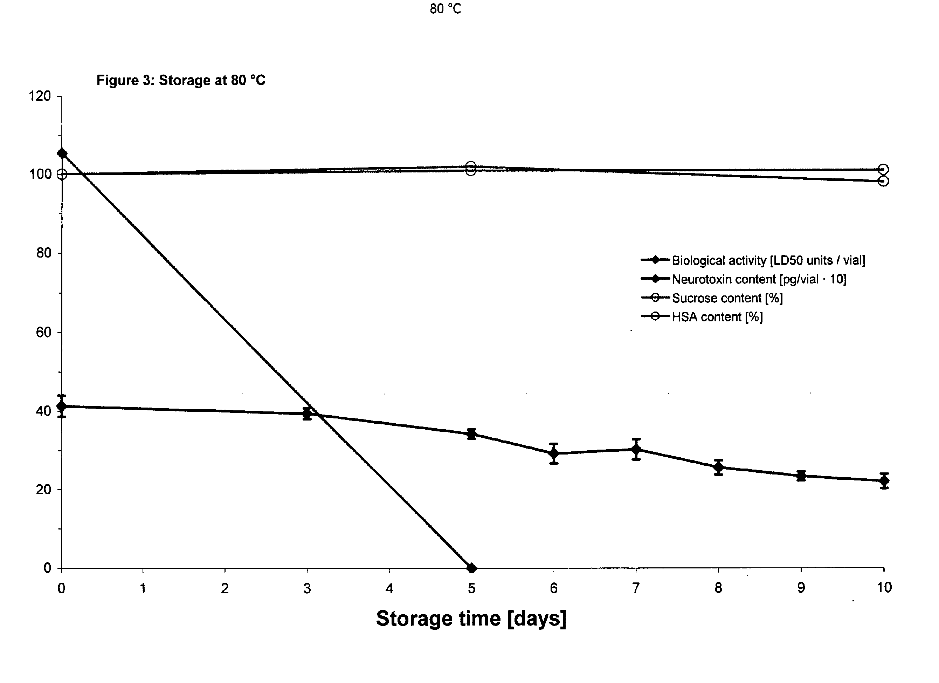

Fig. 3 shows the impact of temperature on the biological activity of Xeomin® after storage at 80°C for a period of up to 10 days; the biological activity was tested at the indicated points in time.

### DETAILED DESCRIPTION OF THE INVENTION

The present invention relates to a process for providing a muscle relaxant at temperatures above 20°C, wherein said muscle relaxant is a solid dry composition comprising the neurotoxic component of botulinum toxin free of complexing agents. Within this invention, the term "providing" includes any kind of provision of the muscle relaxant defined herein, in particular storage, transport, or a step within the preparation of said muscle relaxant. The term "providing" also includes steps wherein the muscle relaxant is subjected to a rise in temperature thereof from the frozen (-20°C) state to a temperature of above 20°C. Within this invention, all forms of the neurotoxin component of botulinum toxin, in particular the various serotypes are to be used. In addition thereto, modified and/or recombinantly produced neurotoxic components of botulinum toxins including the respective mutations, deletions, etc. are also within the scope of the present invention. With respect to suitable mutants, reference is made to WO 2006/027207 A1 and WO 2006/114308 A1, which is fully incorporated by reference herein. Furthermore, within the present invention, mixtures of various serotypes (in the form the neurotoxic component and/or recombinant forms thereof, e.g. mixtures of botulinum neurotoxins of types A and B.

The neurotoxic component referred to herein, may be part of a composition. This composition may contain the neurotoxic component as the sole active component or may contain additional pharmaceutically active components.

Preferably, said composition comprises the neurotoxic component of Botulinum toxin type A. More preferably, said compostion is a lyophilisate of the neurotoxic component of botulinum toxin, even more preferably the composition further comprises sucrose and/or human serum albumin, still more preferably the ratio of human serum albumin to sucrose is about 1:5. In one embodiment, the composition is Xeomin®.

The composition may comprise additional components such as a pH buffer, excipient, cryoprotectant and/or stabilizer.

"pH buffer" refers to a chemical substance being capable to adjust the pH value of a composition, solution and the like to a certain value or to a certain pH range.

"Stabilizing", "stabilizes" or "stabilization" means that the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition.

"Cryoprotectant" refers to excipients which result in the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being freeze-dried in the pharmaceutical composition.

Examples of such stabilizers are gelatin or albumin, preferably of human origin or obtained from a recombinant source. The stabilizers may be modified by chemical means or by recombinant genetics. In a preferred embodiment of the present invention, it is envisaged to use alcohols, e.g., inositol, mannitol, as cryoprotectant excipients to stabilize the neurotoxic component during lyophilization.

In another embodiment, the stabilizer may be a non--proteinaceous stabilizing agent comprising hyaluronic acid or polyvinylpyrrolidone or polyethyleneglycol or a mixture of two or more thereof. Such composition is considered to be a safer composition possessing remarkable stability.

In a more preferred embodiment of the present invention, the composition may comprise the neurotoxic component and a hyaluronic acid or a polyvinylpyrrolidone or a polyethleneglycol, such composition being optionally pH stabilized by a suitable pH buffer, in particular by a sodium acetate buffer, and / or a cryoprotectant polyalcohol.

Preferably, the neurotoxic component has a biological activity of 50 to 250 LD₅₀ units per ng neurotoxic component, as determined in a mouse LD₅₀ assay. More preferably, the neurotoxic component has a biological activity of about 150 LD₅₀.

In another preferred embodiment, the composition may contain a polyalcohol as cryoprotectant. Examples of polyalcohols that might be used include, e.g., inositol, mannitol and other non-reducing alcohols.

Some embodiments of the composition do not comprise a proteinaceous stabilizer, preferably do not contain trehalose or maltotriose or related sugar or polyhydroxy compounds which are sometimes used as cryoprotectants.

The polyvinylpyrrolidone when present in the instant composition, is preferably combined with the instant neurotoxic component in such a quantity to provide a reconstituted solution comprising 10 to 500 mg, especially 100 mg polyvinylpyrrolidone per ml in a 200 U/ml botulinum toxin solution. More preferably, the resulting solution also contains a 1-100 mM, especially 10 mM sodium acetate buffer.

The polyethyleneglycol in the composition is preferably combined with the instant neurotoxic component in such a quantity to provide a reconstituted solution comprising 10 to 500 mg, especially 100 mg polyethyleneglycol per ml in a 200 U/ml botulinum toxin solution. More preferably, the resulting solution also contains a 1-100 mM, especially 10 mM sodium acetate buffer.

Thus, in a preferred embodiment the neurotoxic component is formulated together with a hyaluronic acid stabilizer or a polyvinylpyrrolidone stabilizer or a polyethyleneglycol stabilizer. Additionally, the composition may contain a sodium acetate buffer system and/or an alcoholic cryoprotectant.

Typically, the above referenced provision of the muscle relaxant involves storage and/or transport of the same, or is a step within a process for preparing said muscle relaxant, more preferably a step carried out after the proteins including the neurotoxic component of botulinum toxin have been dried, at elevated temperatures, respectively. By "elevated temperatures" temperatures above 20°C, preferably above 25°C, more preferably 30°C are meant. In exceptional cases, i.e. in an environment where the muscle relaxant on the basis of the neurotoxic component of botulinum toxin is stored below 0°C, the term "elevated temperatures" refers to temperatures above 0°C, preferably above 4°C, more preferably above 10°C, and most preferably to the above recited temperature ranges of above 20, 25 and 30°C, respectively.

In a preferred embodiment, the muscle relaxant is subjected to a temperature lying in the range of above 30°C and up to 70°C for a time period not exceeding 90 days. As the person skilled in the art is perfectly aware of, the time period for which the muscle relaxant is subjected to the respective temperature can be any time interval between a few minutes and 90 days. Typically, taking into account the circumstances of providing such a muscle relaxant, and in particular the situation where storing and/or transportation is involved the time period will not be less than 10 minutes. These short periods are particularly important under circumstances, wherein after transportation and before storage in a hot climate, the muscle relaxant is subjected to direct sunlight, e.g. on an airport or on the street. Typical time periods within the present invention are therefore, 10 minutes, 30 minutes, 1 hour, 3 hours, 4 hours, 5 hours, 6 hours, 7 hours, 8 hours, 1 days, 2 days, 3 days, 4 days, 5 days, 6 days, 7 days, 8 days, 9 days, 10 days, 2 weeks, 3 weeks, 1 month, 2 months, 3 months (90 days). Needless to say, that the time periods mentioned above are only typical examples and the actual time periods may be longer or shorter and include any interval between the numerical values given above.

As to the temperature, to which the muscle relaxant is subjected, typically a lower limit of a temperature of above 20°C is envisioned by the person skilled in the art. With respect to the temperatures and temperature ranges specified herein, the person skilled in the art understands that the upper temperature to which the muscle relaxant/composition is subjected is preferably not above 70°C. This is, the temperatures to which the muscle relaxant is subjected preferably lie in a range of above 20°C and up to 70°C. Therefore, within the present invention, the muscle relaxant is subjected to a temperature above 20°C, or above 25°C, or above 30°C, or above 35°C, or above 40°C, or above 45°C, or above 50°C, or above 60°C, or above 65°C, to up to 70°C, respectively. Again, any specific temperature between the given values of above 20°C and up to 70°C as well as respective temperature intervals, which may be the result of the environment at which the muscle relaxant is provided, preferably transported and/or stored, lies within the present invention.

The following temperature and time intervals represent preferred embodiments of the present invention. According to a first embodiment the muscle relaxant is subjected to a temperature above 30°C and up to 70°C for a time period not exceeding 90 days, more preferably to a temperature above 30°C and up to 70°C for a time period ranging from 10 minutes to 90 days, more preferably at a temperature of between 40 and 60°C and a time period ranging from 10 minutes to 90 days.

In a further preferred embodiment the time period ranges from 10 minutes to 30 days, while the temperature ranges from above 30°C to up to 70°C, preferably from 40°C to 60°C, more preferably from 50°C to 60°C.

In a further preferred embodiment, representing extreme conditions, the temperature lies in the range of between 65°C and 70°C and the time period for which the muscle relaxant is subjected to said temperature lies in the range of from 10 minutes to 10 days, preferably from 10 minutes to 3 days.

Due to the findings on which the present invention is based, it is now possible to provide a muscle relaxant as outlined above without using a device for artificial cooling. This finding is particularly important for the transportation and/or storage of such a muscle relaxant. Furthermore, the invention is particularly relevant in an environment of elevated temperature, possibly together with an increased humidity.

The present invention is now further exemplified by way of the non-limited examples recited hereinunder.

### EXAMPLES

The examples have been conducted with the commercially available product Xeomin®. Xeomin ® is a lyophilized powder containing botulinum neurotoxin type A (150 kD) as active ingredient. The toxin is present in nicked double chain form, i.e. it contains a heavy and a light chain. The toxin is obtained from *Clostridium botulinum* cultures (strain ATCC 3205). It has been purified to such a degree that it is free of any complexing proteins. Xeomin® further comprises human serum albumin and sucrose.

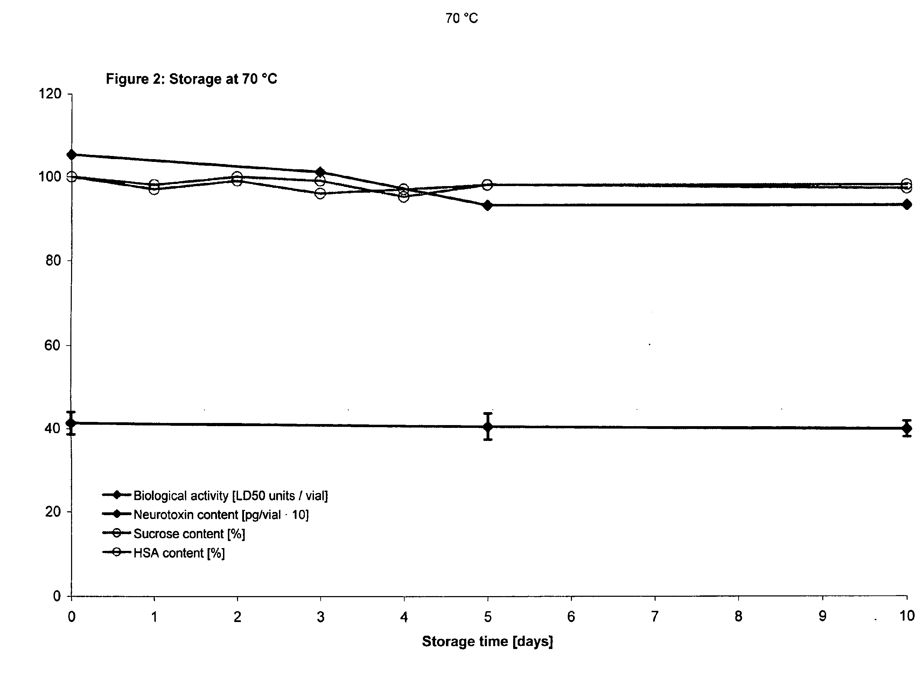

Samples of Xeomin® (unopened vials not reconstituted) were stored at temperatures of 60°C for 30 days (example 1), at 70°C for 10 days (Fig. 2) and 80°C for 10 days (comparative example 3), respectively. The storage was conducted using qualified incubators with narrow temperature tolerance (± 2°C). The samples were consecutively removed from the incubators in daily intervals and stored at 5°C until analysis.

For evaluating the stability of Xeomin®, the biological activity by using the well-known mouse LD₅₀ assay, the content of the neurotoxic component in pg/vial x 10, the sucrose content (in %) and the HSA content (in %) were determined, respectively. The above-mentioned methods were carried out in accordance with the requirements laid down in the European Pharmacopeia.

### EXAMPLE 1:

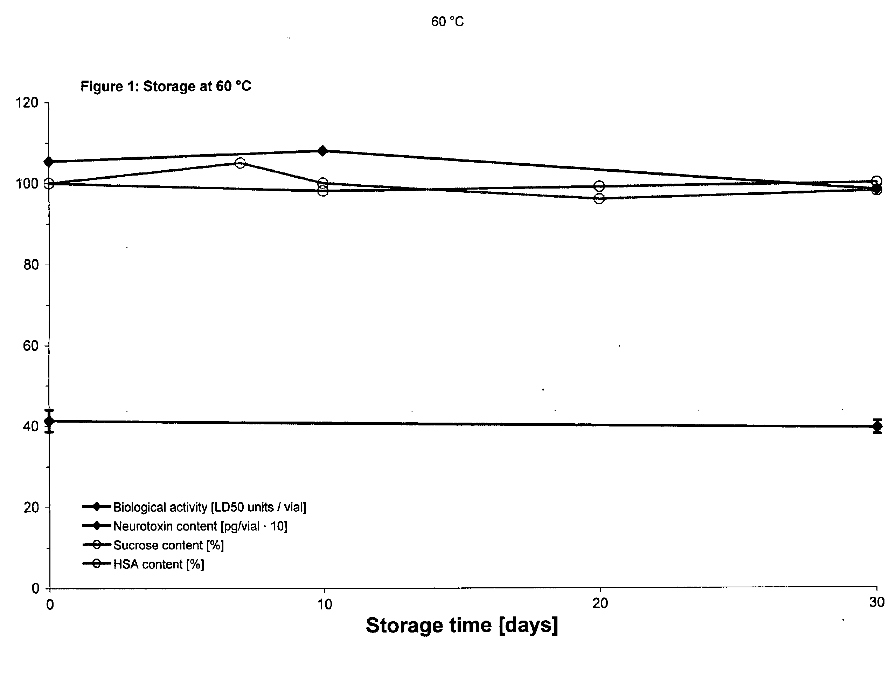

According to this example, Xeomin® was stored for up to 1 month at a temperature of 60°C. The results are shown in Fig. 1.

As it becomes apparent from said figure, the quality of Xeomin® is not affected by storage at 60°C over a period of up to 1 month. Both the biological activity (LD50 assay) and the neurotoxin concentration (ELISA) remain virtually unchanged over the complete time of storage. In addition, the levels of human serum albumin (HSA) and sucrose show no significant variation over time. Taken together, all parameters of the shelf-life specification for Xeomin® are fulfilled after 1 month of storage at 60°C. Similarly, all stability data from storage conditions below 60°C show no detrimental effect on the quality of the product (data not shown).

### EXAMPLE 2:

According to said example, Xeomin® is stored at a temperature of 70°C for a period of up to 10 days. The results are shown in Fig. 2.

The quality of Xeomin® is not significantly affected by storage at 70°C over a period of up to 10 days. Again, the biological activity, the neurotoxin concentration as well as the HSA and sucrose content show no significant variation over time. Taken together, the example demonstrates that the quality of Xeomin® is not significantly affected by the storage at 70°C for up to 10 days.

### EXAMPLE 3 (COMPARATIVE):

In this (comparative) example, Xeomin® is subjected to storage at 80°C for a period of up to 10 days. In contrast to the stability data at 60°C and 70°C, a rapid decrease of the biological activity (LD₅₀ assay) can be observed. The neurotoxin is completely inactivated within 5 days, with a further reduction of activity during the first 3 days of storage. This example shows that at least temperatures of 80°C have a detrimental effect on the stability of a muscle relaxant on the basis of the neurotoxic component of botulinum toxin already after a relatively short period of time.

## Claims

1. Process for providing a muscle relaxant at temperatures above 20°C, wherein said muscle relaxant is a solid dry composition comprising the neurotoxic component of botulinum toxin free of complexing proteins.

2. Process according to claim 1, wherein said provision involves storage and/or transport and/or is a step within a process for preparing said muscle relaxant.

3. Process according to claims 1 or 2, wherein the muscle relaxant is subjected to a temperature above 30°C and up to 70°C for a time period not exceeding 90 days.

4. Process according to any of the preceeding claims, wherein the time period ranges from 10 minutes to 90 days.

5. Process according to any of the preceeding claims, wherein the temperature is between 40 and 60°C and the time period ranges from 10 minutes to 90 days.

6. Process according to any of the preceeding claims, wherein the time period ranges from 10 minutes to 30 days.

7. Process according to any of the preceeding claims, wherein the temperature is between 65°C and 70°C and the time period ranges from 10 minutes to 10 days.

8. Process according to any of claims 2 to 7, wherein said muscle relaxant is transported and/or stored without any device for artificial cooling.

9. Process according to any of the preceeding claims, wherein the composition is a lyophilysate of the neurotoxic component of botulinumtoxin

10. Process according to any of the preceeding claims, wherein the composition further comprises sucrose and/or human serum albumin.

11. Process according to any of the preceeding claims, wherein the composition further comprises at least one component selected from the group consisting of a cryoprotectant, a stabilizer, a pH buffer, an excipient, different from sucrose and human serum albumin, respectively, and mixtures thereof.

12. Process according to any preceedings claims, wherein the neurotoxic component is the neurotoxic component of Botulinum toxin type A.
